# EUROPEAN PATENT APPLICATION

(11) **EP 4 706 677 A1**
(43) Date of publication of application: **11.03.2026**
(21) Application number: 25199126.1
(22) Date of filing: 29.08.2025
(51) Int. Cl.: A61K 40/11, A61K 40/31, A61K 40/42, C12N 15/88

(54) **THERAPEUTIC EFFECT ENHANCER FOR ENHANCING EFFECT OF CAR-T THERAPY**

(30) Priority: 03.09.2024 JP 2024151520
(71) Applicant: KABUSHIKI KAISHA TOSHIBA, Kawasaki-shi, Kanagawa (JP); SHINSHU UNIVERSITY, Matsumoto City, Nagano, 390-8621 (JP)
(72) Inventor: AKAHOSHI, Eiichi, Kawasaki-shi (JP); ISHIHARA, Mitsuko, Kawasaki-shi (JP); NAKAZAWA, Yozo, Matsumoto City, 390-8621 (JP); SAITO, Shoji, Matsumoto City, 390-8621 (JP); NAKASHIMA, Ikumi, Matsumoto City, 390-8621 (JP); YAGYU, Shigeki, Matsumoto City, 390-8621 (JP)
(74) Representative: Marks & Clerk LLP

(57) **Abstract**

According to one arrangement, a therapeutic effect enhancer for promoting a therapeutic effect of a CAR-T cell therapy is provided. The therapeutic effect enhancer includes a nucleic acid that contains a gene encoding a target antigen of a CAR, and a nucleic acid that contains a gene encoding a costimulatory factor.

## Description

### FIELD

The present disclosure relates to a therapeutic effect enhancer for enhancing the effect of CAR-T therapy.

### BACKGROUND

CAR-T cell therapy using T cells (CAR-T cells) genetically modified to produce a chimeric antigen receptor (CAR) that recognizes tumor cells has attracted attention because of its very high therapeutic effect on tumors. In this method, a CAR gene corresponding to a surface antigen of a tumor cell is introduced into T cells collected from a patient to produce CAR-T cells, and the CAR-T cells are administered to the patient. When the administered CAR-T cells encounter a tumor cell having a CAR antigen on its surface in the body of the patient, the CAR-T cells kill the tumor cell.

However, it is also known that the therapeutic effect of CAR-T cell therapy gradually decreases from the start of treatment, and the therapeutic effect tends not to last. This is attributed to the fact that the proportion of tumor cells that have lost the CAR antigen on the cell surface gradually increases, and recognition and killing by CAR-T cells are avoided.

Therefore, there is a need for a method for continuously exerting the effect of the CAR-T cell therapy as compared with the conventional methods.

### BRIEF DESCRIPTION OF THE DRAWINGS

FIG. 1 is a schematic view illustrating a configuration of a therapeutic effect enhancer of a first arrangement.
FIG. 2 is a schematic view illustrating a configuration of a therapeutic effect enhancer of a modification of the first arrangement.
FIG. 3 is a flowchart illustrating a treatment method according to a second arrangement.
FIG. 4 is a graph illustrating an experimental result of Example 1.
FIG. 5 is schematic diagrams for briefly describing an experimental design of Example 2.
FIG. 6 is a graph illustrating an experimental result of Example 2.
FIG. 7 is a graph illustrating an experimental result of Example 3.
FIG. 8 is schematic diagrams for briefly describing an experimental design of Example 4.
FIG. 9 is a graph illustrating an experimental result of Example 4.

### DETAILED DESCRIPTION

According to one arrangement, -----

Hereinafter, a therapeutic effect enhancer of the arrangements and a method for promoting a CAR-T cell therapy using the therapeutic effect enhancer will be described with reference to the drawings. The drawings are schematic diagrams provided to illustrate the arrangements and to facilitate understanding thereof. Accordingly, the shapes, dimensions, proportions, and the like in the drawings may differ from actual configurations. These aspects can be appropriately modified in design with reference to the following description and known techniques.

### [First arrangement]

### - Therapeutic effect enhancer

The therapeutic effect enhancer of the first arrangement is a composition used for enhancing the therapeutic effect of the CAR-T cell therapy, and contains a nucleic acid (hereinafter referred to as "CAR antigen nucleic acid") containing a gene encoding a target antigen of a CAR and a nucleic acid (hereinafter referred to as "costimulatory factor nucleic acid") containing a gene encoding a costimulatory factor.

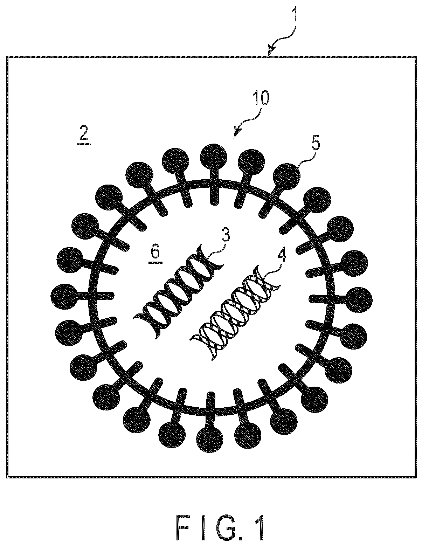

As illustrated in FIG. 1, a therapeutic effect enhancer 1 is desirably provided as a composition in which a lipid particle 10 is contained in a carrier 2. The carrier 2 is, for example, water, a saline solution such as physiological saline, an aqueous glycine solution, or a buffer solution such as HEPES. In addition, the lipid particle 10 desirably encapsulates a CAR antigen nucleic acid 3 and a costimulatory factor nucleic acid 4 in a lipid membrane 5 and has targeting capability to tumor cells.

Hereinafter, each component will be described in detail.

### (Lipid particles)

The lipid particle 10 contained in the therapeutic effect enhancer 1 of the present arrangement is a substantially spherical hollow body including a lipid membrane 5 formed by arranging a plurality of lipid molecules in a non-covalent manner, that is, a liposome. Then, the CAR antigen nucleic acid 3 and the costimulatory factor nucleic acid 4 are encapsulated in the lumen of the lipid particle 10. The lipid membrane 5 may be a lipid monomolecular membrane or a lipid bilayer membrane. In addition, the lipid membrane 5 may be formed of a single layer membrane or may be formed of a multi-layer membrane.

The material of the lipid membrane 5 may include a base lipid illustrated below, and it is preferable that the material of the lipid membrane 5 further contains a lipid compound illustrated below in addition to the base lipid.

As the base lipid, for example, a lipid which is a main component of a biological membrane can be used. The base lipid is a phospholipid or a sphingolipid, such as diacylphosphatidylcholine, diacylphosphatidylethanolamine, ceramide, sphingomyelin, dihydrosphingomyelin, kephalin, or cerebroside, or a combination thereof.

Examples of base lipids preferably used include
1,2-dioleoyl-sn-glycero-3-phosphoethanolamine (DOPE),
1,2-stearoyl-sn-glycero-3-phosphoethanolamine (DSPE),
1,2-dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammonium-propane (DOTMA),
1,2-dioleoyl-3-dimethylammonium-propane (DODAP),
1,2-dimyristoyl-3-dimethylammonium-propane (14:0 DAP),
1,2-dipalmitoyl-3-dimethylammonium-propane (16:0 DAP),
1,2-distearoyl-3-dimethylammonium-propane (18:0 DAP),
N-(4-carboxybenzyl)-N,N-dimethyl-2,3-bis(oleoyloxy)propane (DOBAQ),
1,2-dioleoyl-3-trimethylammonium-propane (DOTAP),
1,2-dioleoyl-sn-glycero-3-phosphocholine (DOPC),
1,2-dilinoleoyl-sn-glycero-3-phosphocholine (DLPC),
1,2-dioleoyl-sn-glycero-3-phospho-L-serine (DOPS), and
cholesterol, or any combination thereof.

In particular, DOTAP is a cationic lipid, and is preferable because the acid dissociation constant of the lipid membrane 5 and thus the lipid particle 10 can be adjusted by varying the content of DOTAP. The base lipid is preferable because the base lipid is easy to fuse with a cell membrane and allows easy control of the structure and particle size of the lipid particle 10. The length of the hydrocarbon chain of the acyl group contained in the lipid is preferably C10 to C20. The hydrocarbon chain may be a saturated hydrocarbon group or an unsaturated hydrocarbon group.

The base lipid may be contained in an amount close to 100% relative to the total lipid molecules contained in the lipid membrane 5, and it is preferable that the base lipid is contained in the lipid membrane 5 in an amount of about 30% to about 80% (in molar ratio) relative to the total lipid molecules.

The lipid compound other than the base lipid is, for example, a biodegradable lipid. Specifically, biodegradable lipids represented by the formula Q-CHR₂ (hereinafter referred to as "lipid compound A") can be used.

Here,
Q is a nitrogen-containing aliphatic group containing two or more tertiary nitrogen and no oxygen,
Rs are each independently an aliphatic group of C12 to C24, and
at least one of the Rs contains, in its main chain or side chain, a linking group LR selected from the group consisting of -C(=O)-O-, -O-C(=O)-, -O-C(=O)-O-, -S-C(=O)-, -C(=O)-S-, -C(=O)-NH-, and -NHC(=O)-.

When the lipid membrane 5 contains the lipid compound A, the cationicity of the surface of the lipid particle 10 can be adjusted, and thus, the disorder in cell introduction is reduced, and the introduction rate of nucleic acid can be enhanced. In addition, when the lipid membrane 5 containing the lipid compound A is used, the encapsulation amount of nucleic acid can be increased, and thus, the introduction rate of nucleic acid can be enhanced.

In particular, it is preferable to use a lipid having a structure represented by the following formula (1-01) and/or (1-02) as the lipid compound A because the efficiency of introducing a nucleic acid into a tumor cell is improved. That is, inclusion of a lipid of the following formula (1-01) and/or (1-02) in the lipid membrane is preferable because the lipid particles can have targeting capability to a tumor cell. In the following description, the lipid of formula (1-01) is referred to as FFT10, and the lipid of formula (1-02) is referred to as FFT20.

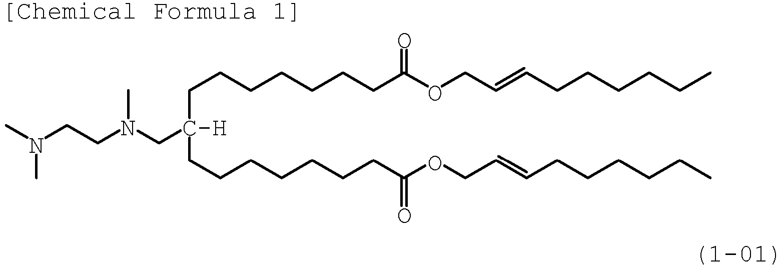

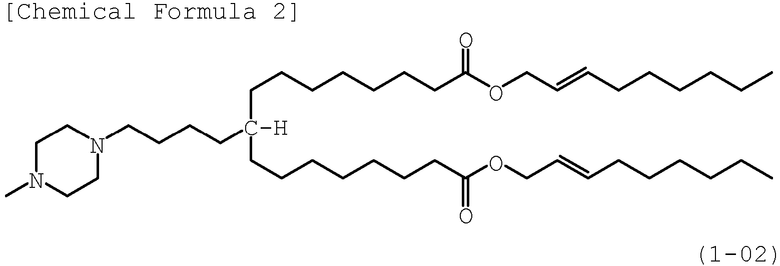

The lipid membrane 5 preferably further contains a lipid (hereinafter referred to as aggregation-reducing lipid) that suppresses aggregation by adjusting the surface charge of the lipid particle 10. Such a lipid is preferably, for example, a PEG-modified lipid, particularly polyethylene glycol dimyristoyl glycerol (DMG-PEG), maleimide-DMG-PEG, polyamide oligomer derived from omega-amino (oligoethylene glycol) alkanoic acid monomer (US 6,320,017), and monosialoganglioside.

As another lipid, the lipid membrane 5 may contain a structure-forming lipid. Examples of the structure-forming lipid include a lipid having relatively low toxicity for adjusting toxicity, a lipid having a functional group that binds a ligand to the lipid particle 10, and a lipid sterol (for example, cholesterol) for suppressing leakage of an encapsulated substance. In particular, cholesterol is preferably contained in the lipid membrane 5 as the structure-forming lipid.

As described above, it is preferable that the lipid membrane 5 of the lipid particle 10 further contains at least one or more lipid compounds from the group consisting of cationic lipids, neutral lipids, aggregation-reducing lipids, and structure-forming lipids in addition to the FFT10 and/or the FFT20 because the nucleic acid encapsulation amount and the nucleic acid introduction efficiency are particularly superior.

### (Nucleic acid)

The CAR antigen nucleic acid 3 and the costimulatory factor nucleic acid 4 are, for example, single-stranded or double-stranded cyclic, linear or branched nucleic acids. The CAR antigen nucleic acid 3 and the costimulatory factor nucleic acid 4 are, for example, any of DNA, RNA, PNA, or derivatives thereof. The derivative is one obtained by inserting a nucleotide analog into a DNA, RNA or PNA, one obtained by modifying the end of any of a DNA, RNA or PNA with a label or a functional group, or the like. The CAR antigen nucleic acid 3 and the costimulatory factor nucleic acid 4 may be in any form of, for example, mRNA, plasmid, and polynucleotide.

When the CAR antigen nucleic acid 3 and/or the costimulatory factor nucleic acid 4 is RNA, it is preferable that the CAR antigen nucleic acid 3 and/or the costimulatory factor nucleic acid 4 is modified to have degradation resistance. For example, the modification may be a known modification that prevents RNA from being degraded by RNase or the like. Such a modification includes, for example, the use or introduction of a modified nucleotide or an artificial nucleotide into RNA, the use or addition of non-natural sequences, or the addition of natural or non-natural CAP structures.

Examples of the modified nucleotide include pseudouridine, 5-methylcytidine, 1-methylpseudouridine, 5-methoxyuridine, and 1-methyladenosine. Examples of the artificial nucleotide includebridged nucleic acid (BNA), locked nucleic acid (LNA), andpeptide nucleic acid (PNA).

The non-natural sequence is, for example, an artificially produced non-naturally occurring base sequence, and is, for example, a random base sequence, a hybrid sequence of a natural/non-natural amino acid and a nucleic acid, or the like. The non-natural sequence is preferably added, for example, to the end of the RNA.

The natural CAP structure is, for example, CAPO (m7GpppN), CAP1 (m7GpppNm), or the like. The non-natural CAP structure is, for example, anti-reverse cap analog (ARCA) or LNA-guanosine. The non-natural CAP structure is preferably added, for example, to the 5' end of the RNA.

The CAR antigen nucleic acid 3 contains a gene encoding a target antigen of the CAR as described above. The target antigen of the CAR in the present disclosure is a protein, a sugar chain, or a glycolipid that is expressed on the surface of a tumor cell and binds to a CAR-T cell. A gene encoding a protein generally known as a CAR antigen may be used as a CAR antigen gene, or a protein modified within a range having a similar function, a fusion protein to which an additional domain is added, a gene encoding a novel protein having a similar function, or the like may be used.

Here, an example of the CAR in the present disclosure will be described in detail. The CAR includes, for example, an extracellular domain, a transmembrane domain, and an intracellular domain.

### (a) Extracellular domain

The extracellular domain is arranged on the outer surface of the cell membrane of the T cell and specifically binds to the target of the CAR. For example, the extracellular domain contains an antigen-binding fragment of an anti-target monoclonal antibody, e.g., a scFv fragment. As the monoclonal antibody, for example, a rodent (mice, rats, rabbits, etc.) antibody, a human antibody or a humanized antibody, an antigen-binding site thereof, or the like can be used. A scFv fragment is a structure in which a light chain variable region (VL) and a heavy chain variable region (VH) of an immunoglobulin are linked via a linker. As the linker, for example, a peptide linker including a peptide in which amino acids are linearly linked can be used. The peptide linker is, for example, a linker (for example, a GGS linker or GS linker) including glycine and serine. For example, a linker having 5 to 25 amino acid residues can be used. Alternatively, when an antigen specific to a tumor cell that is a target of the CAR is a receptor, a ligand that binds to the receptor or the like may be used as the extracellular domain.

The type and configuration of the extracellular domain are selected depending on the type of target of the CAR. The target of the CAR in the present arrangement is the CAR antigen described above.

### (b) Transmembrane domain

The transmembrane domain is a domain between the extracellular domain and the intracellular signal domain in the cell membrane of the T cell. As the transmembrane domain, CD28, CD3ε, CD8α, CD3, CD4, 4-1BB, or the like can be used. Alternatively, it is also possible to use a transmembrane domain including an artificially constructed polypeptide.

### (c) Intracellular signal domain

The intracellular signal domain is arranged inside the cell membrane of the T cell, and transmits a signal necessary for activation of the T cell when the extracellular domain binds to the target antigen. The intracellular signal domain includes, for example, a domain (first domain) for transmitting a signal via a TCR complex. As the first domain, CD3ζ, FcεRIγ, or the like can be used. Preferably, CD3ζ is used.

The intracellular signal domain may further include a domain (second domain) for transmitting a costimulatory signal. As the second domain, for example, an intracellular domain of a costimulatory molecule such as CD28, 4-1BB (CD137), CD2, CD4, CD5, CD134, OX-40, or ICOS can be used. Preferably, CD28 or 4-1BB is used.

Each of the first domain and the second domain may include one of the elements listed above, or may have a configuration in which a plurality of the same or different elements listed above are linked in a tandem manner. The order in which the first domain and the second domain are linked is not particularly limited, but it is preferable to arrange the second domain on the transmembrane domain side. The first domain and the second domain may be directly linked, or a linker may be interposed therebetween. As the linker, for example, a peptide linker including a peptide in which 2 to 15 amino acids are linearly linked can be used.

### (d) Other elements

The CAR may include other elements. As other elements, for example, a leader sequence (signal peptide) that promotes CAR secretion, a leader sequence of GM-CSF receptor, or the like can be used. A spacer domain may be arranged between the extracellular domain and the transmembrane domain. The spacer domain may facilitate binding of the CAR to the target. As the spacer domain, for example, an Fc fragment of human IgG (for example, human IgG1, human IgG4) can be used. Alternatively, a part of the extracellular domain of CD28, a part of the extracellular domain of CD8α, or the like can be used as the spacer domain. The spacer domain can also be provided between the transmembrane domain and the intracellular signal domain.

The CAR gene is, for example, a gene corresponding to each of the above domains bound in an appropriate order. A CAR gene sequence may include the entire length of any of the above-described CAR genes, or may include a part thereof.

The CAR antigen in the present disclosure is typically an antigen associated with a tumor cell or an antigen specific to a tumor. The antigen associated with the tumor cell refers to an antigen that exhibits significant or notable expression (i.e., specific or selective expression), for example, in tumor cells, in cells of surrounding tissues, or in a subject having tumor cells, as compared to non-tumor cells. The CAR antigen may be an antigen known as a substance that is a target of the CAR (that is, compatible with the type of the CAR of interest) and generally cannot be confirmed by normal cells. The tumor-associated antigen or the tumor-specific antigen may be an antigen present in an extracellular matrix, or a protein containing a mutation identified by genome analysis and/or a suggestive expression study of a tumor, etc.

When the above tumor is, for example, B-cell lymphoma, multiple myeloma, retinoblastoma, pulmonary alveolar tumor, or central nervous system tumor, the CAR antigen may include, for example, CD19 antigen, CD20 antigen, GD2 antigen, CD22 antigen, CD30 antigen, CD33 antigen, CD44 variant 7/8 antigen, CEA antigen, Her2/neu antigen, MUC1 antigen, MUC4 antigen, MUC6 antigen, IL-13 receptor-alpha2, immunoglobulin light chain, PSMA antigen, or VEGF receptor 2. Alternatively, when the tumor is a leukemia stem cell, a leukemia precursor cell, a leukemia cell, or the like of a myeloid tumor, it is also possible to use GM-CSF that is a ligand of a GM-CSF (granulocyte monocyte colony stimulating factor) receptor as the CAR antigen. Alternatively, in the case of neuroblastoma, breast cancer, cervical cancer, uterine cancer, ovarian cancer, melanoma, astrocytoma, Ewing's sarcoma, glioblastoma, retinoblastoma, rhabdomyosarcoma, non-small cell lung cancer, prostate cancer, or urothelial cancer, it is also possible to use FAM150A, FAM150B and fragments thereof that bind to the extracellular ligand-binding regions of ALK as the CAR antigen.

In addition, depending on the type of tumor of interest, the CAR antigen may also be an antigen such as EphB4, EGFR variant 3, EphA2, EphB2, EGFR, GD2, Glypican-3, 5T4, 8H9, αvβ6 integrin, B cell maturation antigen (BCMA), B7-H3, B7-H6, CAIX, CA9, κ light chain, CD38, CD44, CD44 variant 6, CD70, CD116, CD123, CD138, CD171, CEA, CSPG4, EGP2, EGP40, EPCAM, ERBB3, ERBB4, ErbB3/4, FAP, FAR, FBP, fetal AchR, folate receptor α, GD3, HLA-A1, MAGE A1, HLA-A2, IL11Ra, IL13Ra2, KDR, lambda, Lewis Y, MCSP, mesothelin, NCAM, NKG2D ligand, NY-ESO-1, PRAME, PSCA, PSC1, ROR1, Sp17, SURVIVIN, TAG72, TEM1, TEM8, VEGR receptor 2, carcinoembryonic antigen, HMW-MAA, VEGF receptor, fibronectin, tenascin, ALK (anaplastic lymphoma kinase), or an antigen present in an extracellular matrix such as CEA in the necrotic region of a tumor. Furthermore, the CAR antigen may be a full length or a part of a base sequence obtained by combining a plurality of these genes.

The costimulatory factor nucleic acid 4 contains a gene encoding a costimulatory factor as described above. The "costimulatory factor" in the present disclosure is a protein that is naturally expressed on a membrane of an antigen-presenting cell such as a dendritic cell, and transmits an auxiliary signal for activation to a T cell by binding to and interacting with a costimulatory receptor on the T cell. The costimulatory factor is also generally referred to as "costimulatory molecule", "secondary stimulatory factor", or "secondary stimulatory molecule".

As the costimulatory factor nucleic acid 4 in the present disclosure, a gene encoding a protein generally known as a costimulatory factor can be used as the costimulatory factor nucleic acid. Specifically, the costimulatory factor nucleic acid 4 may be a nucleic acid encoding at least a costimulatory factor selected from the group consisting of 4-1-BBL, CD80, CD86, ICOSL, and OX40L. Alternatively, a gene encoding a protein modified within a range having functions similar to these, a fusion protein to which an additional domain is added, or a novel protein having similar functions, or the like may be used.

### (Nucleic acid condensing peptide)

The nucleic acid condensing peptide is a peptide capable of further reducing the volume occupied by nucleic acid by condensing more nucleic acid, and can efficiently encapsulate more nucleic acid in the lipid particle 10. As such a peptide, for example, a cationic peptide is preferably used. The cationic peptide can condense the nucleic acid, for example, by entering into a spiral gap of the anionic nucleic acid to shorten the gap.

A preferred nucleic acid condensing peptide is, for example, a peptide containing cationic amino acids in an amount of 45% or more of the total. A more preferred nucleic acid condensing peptide has RRRRRR (first amino acid sequence) at one end and has a sequence RQRQR (second amino acid sequence) at the other end. Then, between the two amino acid sequences, 0 or 1 or more intermediate sequences including RRRRRR or RQRQR are contained. In addition, two or more neutral amino acids are contained between two adjacent sequences among the first amino acid sequence, the second amino acid sequence, and the intermediate sequence. The neutral amino acid is, for example, G or Y.

The nucleic acid condensing peptide preferably has the following amino acid sequence.
RQRQRYYRQRQRGGRRRRRR (SEQ ID NO: 1)
RQRQRGGRRRRRR (SEQ ID NO: 2)

According to such a nucleic acid condensing peptide, it is possible to efficiently condense the nucleic acid by arginine that is cationic and to weaken the anionicity of the nucleic acid, and thus, it is possible to efficiently encapsulate the nucleic acid in the lipid particle 10. Furthermore, since the nucleic acid condensing peptide efficiently dissociates the nucleic acid in the cell, the CAR antigen nucleic acid 3 and the costimulatory factor nucleic acid 4 introduced into the cell can be efficiently expressed in the tumor cell.

Alternatively, the nucleic acid condensing peptide has RRRRRR (third amino acid sequence) at one end and RRRRRR (fourth amino acid sequence) at the other end. Then, between the two amino acid sequences, 0 or 1 or more intermediate sequences including RRRRRR or RQRQR are contained. In addition, two or more neutral amino acids are contained between two adjacent sequences among the third amino acid sequence, the fourth amino acid sequence, and the intermediate sequence.

Such a nucleic acid condensing peptide preferably has the following amino acid sequence.
RRRRRRYYRQRQRGGRRRRRR (SEQ ID NO: 3)

Such a nucleic acid condensing peptide has strong cationicity at both ends and high binding to nucleic acid. Therefore, it is possible to more efficiently condense the nucleic acid and encapsulate more nucleic acid in the lipid particle 10. As a result, the amount of nucleic acid remaining outside the lipid particle 10 is reduced, thereby preventing aggregation of the lipid particles 10, and thus facilitating the cellular uptake of the lipid particles 10.

Furthermore, a nucleic acid condensing peptide having the following amino acid sequence can also be used in combination with any of the nucleic acid condensing peptides described above.
GNQSSNFGPMKGGNFGGRSSGPYGGGGQYFAKPRNQGGY(M9) (SEQ ID NO: 4)

This peptide can further condense the nucleic acid aggregate condensed with the nucleic acid condensing peptide. Therefore, it is possible to obtain a lipid particle having a smaller particle size. As the particle size is smaller, the lipid particle is more likely to be taken into a tumor cell, and thus, it is possible to more efficiently introduce the nucleic acid into the genome of the tumor cell. For example, the CAR antigen nucleic acid 3 and the costimulatory factor nucleic acid 4 can be condensed by stirring and mixing the CAR antigen nucleic acid 3 and the costimulatory factor nucleic acid 4 with the nucleic acid condensing peptide before being encapsulated in the lipid particle 10.

It is more preferable to use the nucleic acid condensing peptide from the viewpoint of achieving the above-described effects, but depending on the type of the nucleic acid to be used, the culture conditions of the cell, or the like, it is also possible to omit the use of the nucleic acid condensing peptide.

The lipid particle 10 may encapsulate further components in addition to the CAR antigen nucleic acid 3, the costimulatory factor nucleic acid 4, and the nucleic acid condensing peptide. For example, the lipid particle 10 may contain a compound that modulates expression of a nucleic acid in a cell, such as retinoic acid, cyclic adenosine monophosphate (cAMP), or ascorbic acid. Alternatively, the lipid particle 10 can also encapsulate, for example, peptides, polypeptides, cytokines, growth factors, apoptosis factors, differentiation-inducing factors, other cell surface receptors, ligands thereof, and the like.

The lipid particle 10 can be produced, for example, using a known method used when a small molecule is encapsulated in a lipid particle or the like. Examples of the known method include a bangum method, an organic solvent extraction method, an ethanol injection method, a surfactant removal method, and a freeze-thaw method. When the organic solvent extraction method is selected, for example, an aqueous buffer containing components to be encapsulated such as the CAR antigen nucleic acid 3 and the costimulatory factor nucleic acid 4 is added to a mixture obtained by dissolving the material of the lipid membrane 5 in an organic solvent such as alcohol, and the mixture is stirred and suspended, whereby the lipid particle 10 can be produced. When the ethanol injection method is selected, for example, ethanol obtained by dissolving the material of the lipid membrane 5 is added to an aqueous buffer containing components to be encapsulated such as the CAR antigen nucleic acid 3 and the costimulatory factor nucleic acid 4, and the mixture is stirred and suspended, whereby the lipid particle 10 can be produced. The amount ratio of the nucleic acids encapsulated in the lipid particle 10 can be easily adjusted by changing the amount ratio of the nucleic acids in the aqueous buffer. The encapsulation amount of nucleic acid can be confirmed using, for example, commercially available DNA and RNA quantification kits.

The average particle size of the lipid particle 10 is, for example, from about 50 nm to about 300 nm, preferably from about 50 nm to about 200 nm. For example, the particle size can be reduced by ultrasonic treatment. In addition, it is also possible to adjust the size of the lipid particle 10 by allowing the particle to pass through a polycarbonate membrane or a ceramic membrane. The average particle size of the lipid particle 10 can be measured by, for example, a zetasizer using a dynamic light scattering method.

The therapeutic effect enhancer may further contain a substance that improves the storage stability of the lipid particle, that is, a storage stabilizer. The storage stabilizer is a pH adjuster, a buffering agent, a tonicity adjuster, a cryoprotectant, or the like. Specifically, the storage stabilizer for lipid particles is not limited, and is, for example, a glycoprotein such as albumin, lipoprotein, apolipoprotein, or globulin. Alternatively, the storage stabilizer may be a pharmaceutically acceptable adjusting agent such as sodium acetate, sodium lactate, sodium chloride, potassium chloride, and calcium chloride, serving to bring the pharmaceutical composition closer to physiological conditions. Alternatively, the storage stabilizer may be a fatty affinity free radical quencher such as α-tocopherol that suppresses damage by free radicals. In addition, the storage stabilizer may be a lipid protectant, etc. such as a water-soluble chelator like ferioxamine, which suppresses peroxidative damage of lipids and improves storage stability. The storage stabilizer is preferably added to a solution containing the lipid particle 10 or the like after the formation of the lipid particle 10.

The therapeutic effect enhancer may be sterilized by common methods. The therapeutic effect enhancer may also be provided as a liquid or as a dried powder. In the case of the powdery form, the therapeutic effect enhancer can be used, for example, by being dissolved in an appropriate liquid.

The concentration of the lipid particle 10 contained in the composition is not limited, but is preferably 0.01 to 30 mass%, and more preferably 0.05 to 10 mass%. The concentration is appropriately selected according to the purpose.

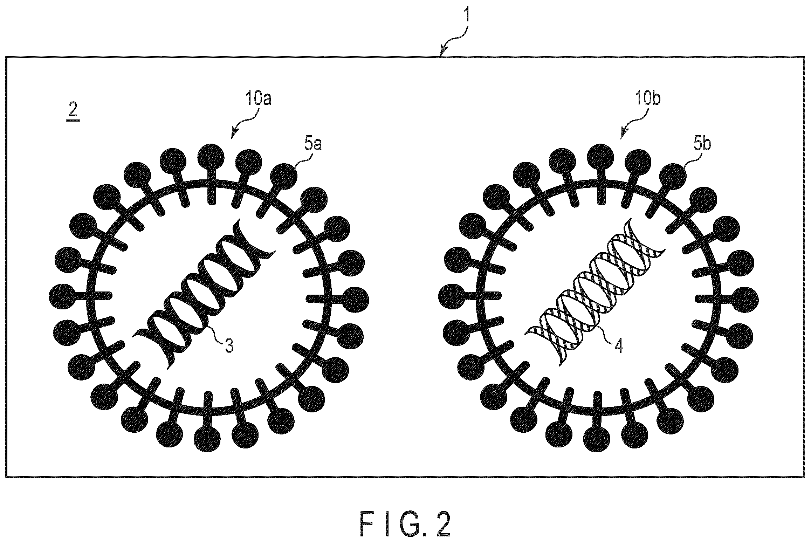

According to a further arrangement, the therapeutic effect enhancer 1 may be provided as a composition in which two kinds of lipid particles including a lipid particle 10a encapsulating the CAR antigen nucleic acid 3 and a lipid particle 10b encapsulating the costimulatory factor nucleic acid 4 are contained in a carrier (see FIG. 2). In this case, the lipid particles 10a and 10b contain, for example, the lipid compound A as a lipid constituting each of lipid membranes 5a and 5b, and particularly contain the FFT10 and/or the FFT20, thereby exhibiting targeting capability to tumor cells. The two kinds of lipid particles 10a and 10b may include the same lipid composition or different lipid compositions as long as the preferable conditions of the lipid composition described in the first arrangement are satisfied. That is, the lipid compositions of the lipid membranes 5a and 5b may be the same or different. The therapeutic effect enhancer 1 can be produced by separately producing the lipid particle 10a and the lipid particle 10b and mixing carriers containing the respective lipid particles.

### [Second arrangement]

### - Methods of treatment to promote therapeutic effects of CAR-T cell therapy

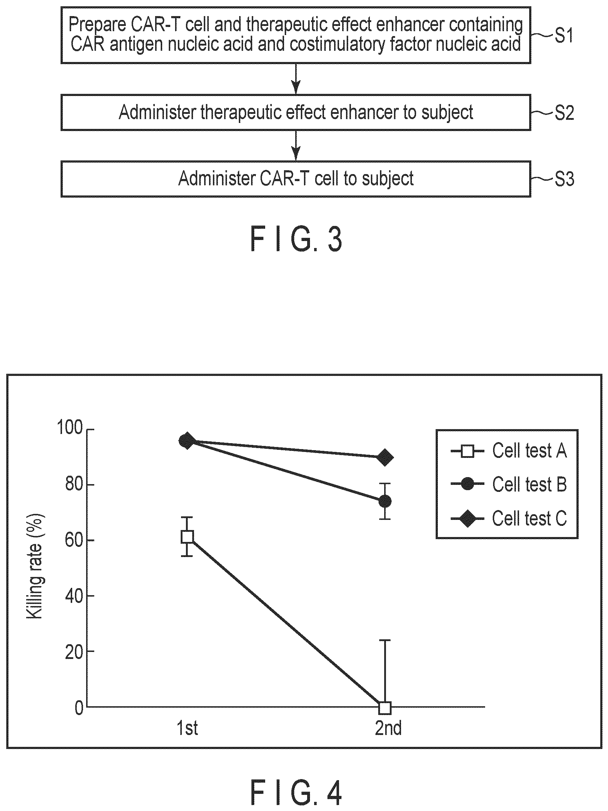

The method of the second arrangement is a treatment method for promoting the therapeutic effect of the CAR-T cell therapy on the subject to be treated, and uses the therapeutic effect enhancer of the first arrangement. An example of the method of the second arrangement will be described below with reference to FIG. 3.

The method of the second arrangement includes a preparation step (S1) of preparing a CAR-T cell and a therapeutic effect enhancer containing a lipid particle encapsulating a CAR antigen nucleic acid and a costimulatory factor nucleic acid; a gene introduction step (S2) of administering the therapeutic effect enhancer to a subject; and a treatment step (S3) of administering the CAR-T cell to the subject.

Here, the tumor cell in the present disclosure refers to a cell constituting an epithelial tumor, a non-epithelial tumor, or a malignant tumor including both epithelial tumor and non-epithelial tumor, and is also generally referred to as "cancer cell" or "carcinoma cell". The term "tumor cell" encompasses tumor cells at any stage of disease progression, including, for example, a state in which the malignant tumor remains within the organ of origin, a state in which the malignant tumor has invaded surrounding tissues, a state in which the malignant tumor has metastasized to lymph nodes, and a state in which the malignant tumor has metastasized to distant organs. The CAR-T cell is designed to express a CAR that binds to a surface antigen of the tumor cell.

The therapeutic effect enhancer prepared in the preparation step (S1) is, for example, a composition containing a lipid particle 10 encapsulating a CAR antigen nucleic acid 3 and a costimulatory factor nucleic acid 4. Alternatively, the therapeutic effect enhancer may be a composition in which two kinds of lipid particles including a lipid particle 10a encapsulating the CAR antigen nucleic acid 3 and a lipid particle 10b encapsulating the costimulatory factor nucleic acid 4 are contained in a carrier.

In the gene introduction step (S2), the contact between the therapeutic effect enhancer and the tumor cell can be achieved by administering the therapeutic effect enhancer prepared in the preparation step (S1) to the subject. When the subject is a living body, cells other than tumor cells are present, but the therapeutic effect enhancer of the present arrangement contains lipid particles having targeting capability to tumor cells, and has little influence on normal cells.

The administration route of the CAR-T cell to the living body is not particularly limited. For example, the CAR-T cell is administered by intravenous injection, intraarterial injection, intraportal injection, intradermal injection, subcutaneous injection, intramuscular injection, or intraperitoneal injection. Alternatively, the administration method may be topical administration. In that case, for example, the CAR-T cell is directly injected into a target tissue, organ, or anatomical site. The administration schedule may be selected in consideration of gender, age, weight, disease state, or the like of the subject (patient). For example, the administration schedule may be a single administration or a plurality of consecutive or regular administrations.

In the gene introduction step (S2), when the lipid particle 10 comes into contact with the tumor cell, the lipid particle 10 is taken into the tumor cell by endocytosis, membrane fusion between the lipid particle and the cell membrane of the tumor cell, or the like. Next, the CAR antigen nucleic acid 3 and the costimulatory factor nucleic acid 4 are released from the lipid particle 10 taken into the tumor cell into the tumor cell, whereby the CAR antigen nucleic acid and the costimulatory factor nucleic acid can be introduced into the tumor cell. Since the introduced CAR antigen nucleic acid expresses the CAR antigen on the surface of the tumor cell, the targetability of the tumor cell in which the surface antigen has disappeared can be restored. In addition, the introduced costimulatory factor nucleic acid is expressed on the surface of the tumor cell.

In order to cause the introduced CAR antigen nucleic acid and costimulatory factor nucleic acid to be expressed, a period from the gene introduction step (S2) to the treatment step (S3) described next may be provided. For example, the next treatment step (S3) may be performed after a period of several days to several weeks following the administration of the therapeutic effect enhancer.

In the treatment step (S3), after the gene introduction step (S2), the CAR-T cell prepared in the preparation step (S1) is administered to the subject. Thus, the CAR-T cell is brought into contact with the tumor cell expressing the CAR antigen and the costimulatory factor in the gene introduction step (S2).

In the treatment step (S3), the CAR-T cell recognizes the CAR antigen expressed on the surface of the tumor cell and kills the tumor cell. Furthermore, since the costimulatory factor is expressed on the surface of the recognized tumor cell, the CAR-T cell is activated by interaction to enhance its tumor cell killing property.

Here, the CAR-T cell may be produced by a general method. As long as a T cell is contained, the CAR-T cell can be produced using various cell populations. For example, the cell population containing the T cell may be peripheral blood mononuclear cells (PBMC) collected from peripheral blood. Alternatively, the CAR-T cell may be produced in a manner that a CD4-positive CD8-negative T cell, a CD4-negative CD8-positive T cell, a T cell prepared from an iPS cell, an αβ-T cell, a γδ-T cell, a precursor cell thereof, or the like expresses the CAR. The cell population containing the T cell is more preferably a cell population collected from a subject to whom the CAR-T cell is administered (that is, an autologous cell population). Here, allogeneic cell populations, commercially available cell populations, or the like may also be used. In addition, a cell population subjected to a treatment of separating unnecessary cells from the collected or obtained cell population may be used.

The introduction of the CAR gene into the T cell may be performed using any tool or method generally known as a gene introduction tool or a gene introduction method for the introduction of gene into the cell. Further, as long as the CAR is expressed, it is not essential whether the CAR gene is integrated into the genome of the T cell or not; however, it is more preferable that the CAR gene is integrated into the genome. For example, polycation, calcium phosphate, lipofection, liposome, electroporation method, microinjection, particle gun, retro/lentiviral vector, adenoviral vector, or the like may be used as the gene introduction tool or the gene introduction method for the introduction of gene into the T cell.

In addition, a transposon vector, a homologous recombination method, a tool using a recombinase system, or a foreign gene integration tool such as a genome editing tool like ZFN or CRISPR/Cas9 may be used in combination.

In the method of the second arrangement, after the treatment step (S3), the same gene introduction step as in (S2) may be further repeated a plurality of times, and at the same time, the same treatment step as in (S3) may be repeated a plurality of times.

Conventionally, there is known a method of introducing only the CAR antigen gene using a virus as a gene introduction method for tumor cells. However, in such a method, administration of the virus to a living body results in the acquisition of immunity against the virus, thereby making it impossible to perform a plurality of administrations, and consequently limiting the treatment to a single administration. Therefore, in the conventional method using a virus, it is difficult to perform continuous treatment. On the other hand, since the method of the present arrangement does not use a virus, it is possible to perform a plurality of administrations, and even when the surface antigen of the tumor cell disappears again during the treatment, the targetability of the tumor cell can be restored again.

The method of the second arrangement can introduce a gene into a tumor cell with high efficiency using a therapeutic effect enhancer, and thus can kill the tumor cell with high efficiency. Although cells other than tumor cells are present in a living body, the therapeutic effect enhancer of the present arrangement is superior in that it contains lipid particles having targeting capability to tumor cells and has little influence on normal cells other than tumor cells.

According to a further arrangement, the timing of dosing the therapeutic effect enhancer may be after initiation of the treatment with the CAR-T cell. That is, the CAR-T cell therapy of the further arrangement may include: a preparation step (S1) of preparing a therapeutic effect enhancer containing a lipid particle encapsulating a CAR antigen nucleic acid and a costimulatory factor nucleic acid, and a CAR-T cell; a treatment step (S2') of administering the CAR-T cell to a subject; and a gene introduction step (S3') of administering the therapeutic effect enhancer to the subject after S2'.

### [Examples]

Hereinafter, an example in which the therapeutic effect enhancer of the arrangement is prepared and used in the method of the arrangement will be described. However, the arrangement of the present invention is not limited to the following examples.

### Example 1. Confirmation of tumor cell cytotoxic effect and persistence of the therapeutic effect enhancer by cell test

In order to confirm the tumor cell cytotoxic effect by the therapeutic effect enhancer and the persistence thereof, the following cell tests A to C were performed.

The cell test A is a comparative example for confirming the tumor cell cytotoxic effect by the CAR-T cell. As the CAR-T cell, an ephrin receptor B4 (EphB4) CAR-T cell was used. The CAR antigen was the extracellular domain of the EphB4 protein. Specifically, in the cell test A, the CAR-T cell was brought into contact with the tumor cell and cultured, and then subculture was further performed, and the tumor cell and the CAR-T cell were brought into contact again.

The cell test B is a comparative example for confirming the tumor cell cytotoxic effect by the CAR-T cell after adding a therapeutic effect enhancer containing no costimulatory factor. Specifically, the CAR-T cell was brought into contact with the tumor cell into which a gene was introduced by a liposome encapsulating an mRNA encoding a CAR antigen (extracellular domain of the EphB4 protein) and cultured, and then subculture was further performed, and the tumor cell and the CAR-T cell were brought into contact again under the same conditions.

The cell test C is an example for confirming the tumor cell cytotoxic effect by the CAR-T cell after adding the therapeutic effect enhancer of the first arrangement. Specifically, the CAR-T cell was brought into contact with the tumor cell into which a gene was introduced by a liposome co-encapsulating an mRNA encoding a CAR antigen and a costimulatory factor (CD80 and 4-1BBL) and cultured, and then subculture was further performed, and the tumor cell and the CAR-T cell were brought into contact again under the same conditions.

### - Preparation of tumor cell

The alveolar rhabdomyosarcoma cell line Rh30, which was suspended in Dulbecco's Modified Eagle Medium (DMEM) supplemented with 10% fetal bovine serum, was seeded at 1 × 10⁵ cells per well in a 4-well culture plate, and placed in an incubator having 5% CO₂ atmosphere at 37°C to prepare a tumor cell.

### - Preparation of CAR-T cell

An operation of introducing a CAR gene into a human PBMC was performed to produce a cell population containing a CAR-T cell. The human PBMC was placed in an incubator having 5% CO₂ atmosphere at 37°C with ALyS705 (containing IL7: 10 ng/mL, IL15: 5 ng/mL) supplemented with 5% artificial serum (Cell Science & Technology Institute, Inc.), and was cultured. The CAR-T cell was produced by a piggyBAC method using a DNA transferase derived from a moth. That is, a plasmid DNA for CAR expression and a plasmid DNA for piggyBAC expression were introduced into the PBMC by an electroporation method to prepare the CAR-T cell.

### - Preparation of lipid particle

As the nucleic acid to be encapsulated in the lipid particle, the mRNA encoding the extracellular domain of the CAR antigen EphB4 was used in the cell test B, and the mRNA encoding the extracellular domain of the CAR antigen EphB4 and the mRNA encoding CD80 and 4-1BBL of costimulatory factors were used in the cell test C. A solution containing each mRNA and an lipid-dissolved ethanol solution having a lipid composition described in the following Table 1 were mixed, then further mixed with 10 mM HEPES (pH7.3), and washed and concentrated by centrifugal ultrafiltration to obtain respective solutions containing lipid particles used for the cell tests B and C. [Table 1]

**Table 1**

| Lipid | FFT10 | FFT20 | DOPE | DOTAP | DMG-PEG | Cholesterol |
|---|---|---|---|---|---|---|
| Composition | 0 | 32 | 9 | 4.5 | 52 | 3.5 |

| | | | | | | |
|---|---|---|---|---|---|---|
| (Unit: %, molar fraction) | | | | | | |

### - Gene introduction into tumor cell by lipid particle

In the cell tests B and C, each lipid particle was brought into contact with a tumor cell to perform gene introduction into the tumor cell. Specifically, each lipid particle-containing solution prepared as described above was added to a DMEM medium supplemented with 10% fetal bovine serum containing the tumor cell Rh30 prepared as described above to introduce the gene. After mixing with the lipid particle-containing solution, the mixture was cultured for one day to express the gene.

### - Measurement of cytotoxic effect of CAR-T cell on tumor cell

In the cell test A, after preparation of the tumor cell Rh30 and the EphB4 CAR-T cell, a medium containing both was mixed, and the survival rate of the tumor cells after 5 days, that is, the killing rate by the EphB4 CAR-T cell was measured. In the cell tests B and C, after the expression of the transgene in the tumor cell Rh30 and the preparation of the EphB4 CAR-T cell were completed, both medium containing the Rh30 and EphB4 CAR-T cell were mixed, and the survival rate of the tumor cells after 5 days, that is, the killing rate by the EphB4 CAR-T cell was measured. The killing rate (%) of the tumor cells by the CAR-T cell was calculated by dividing the number of tumor cells in each condition by the number of negative control tumor cells (the number of tumor cells cultured without adding the CAR-T cell) and then multiplying the obtained value by 100. Further, in all the cell tests A, B, and C, the tumor cell that survived after the measurement of the killing rate was brought into contact with a new lipid particle to be brought into contact with a new CAR-T cell into which the gene was introduced, and the killing rate by the CAR-T cell was measured.

### - Results

The experimental results of Example 1 are illustrated in FIG. 4. In FIG. 4, the numerical value on the vertical axis indicates the killing rate, "1st" on the horizontal axis indicates the result of the first killing rate measurement, and "2nd" on the horizontal axis indicates the result of the second killing rate measurement after subculture.

As illustrated in FIG. 4, in the cell test A, which is a control test, the first killing rate was about 60%, but the second killing rate was very low. This confirmed that the tumor cell Rh30 has bypassed the immune system. In addition, in the cell test B, a killing rate of slightly more than 90% was confirmed in the first measurement, but a killing rate in the second measurement was about 70%, and thus, it can be seen that the cytotoxic effect on the tumor cell Rh30 by the EphB4 CAR-T cell was reduced.

On the other hand, in the cell test C, the killing rate in the first measurement was slightly more than 90%, and the killing rate in the second measurement was also about 90%, and thus, the persistence of the cytotoxic effect by the EphB4 CAR-T cell was confirmed even on the tumor cell Rh30 capable of avoiding the immune system. Furthermore, it was confirmed that the EphB4 CAR-T cell acquired a sustained tumor cell cytotoxic effect by being activated by contact with the tumor cell Rh30 expressing a CAR antigen and a costimulatory factor. From the above results, it was illustrated that the expression of the costimulatory factors CD80 and 4-1BBL is effective in maintaining the cytotoxic effect of the CAR-T cell on tumor cells.

### Example 2. Confirmation of tumor cell cytotoxic effect of therapeutic effect enhancer by animal test

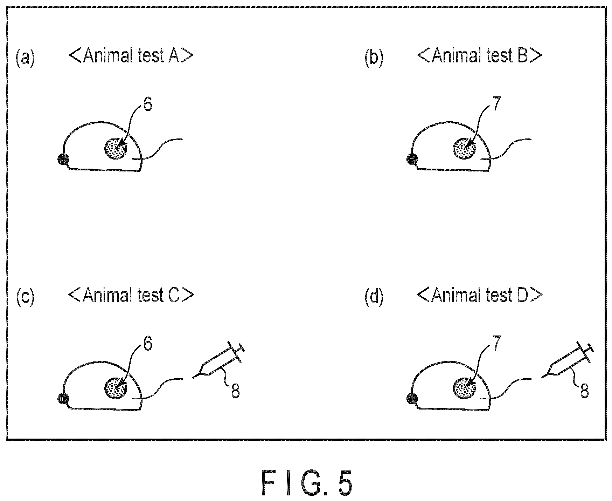

In order to confirm the tumor cell cytotoxic effect depending on the presence or absence of costimulatory factors by animal tests, the following animal tests A to D were performed. First, the experimental designs of the animal tests A to D will be briefly described with reference to FIG. 5.

As illustrated in FIG. 5, the animal test A is a comparative example for confirming the proliferation rate of untreated tumor cells. Specifically, a tumor cell 6 originally expressing a small amount of CAR antigen as an untreated tumor cell was transplanted into a mouse, and the proliferation rate was measured over time.

As illustrated in FIG. 5, the animal test B is a comparative example for confirming the proliferation rate of tumor cells expressing costimulatory factors in an untreated case. Specifically, the tumor cell 6 originally expressing a small amount of CAR antigen was treated with the therapeutic effect enhancer of the first arrangement to produce a costimulatory factor-expressing tumor cell 7, the costimulatory factor-expressing tumor cell 7 was transplanted into a mouse, and the proliferation rate of the costimulatory factor-expressing tumor cell 7 was measured over time.

As illustrated in FIG. 5, the animal test C is a comparative example for confirming the cytotoxic effect of a CAR-T cell 8 on untreated tumor cells. Specifically, the tumor cell 6 expressing a small amount of CAR antigen was transplanted into a mouse, and the CAR-T cell 8 was further administered to the mouse to measure the proliferation rate of the tumor cell 6 over time.

As illustrated in FIG. 5, the animal test D is an example for confirming the cytotoxic effect of the CAR-T cell on the tumor cell which is induced to express a costimulatory factor by the therapeutic effect enhancer of the first arrangement. Specifically, the costimulatory factor-expressing tumor cell 7, which was produced by treating the tumor cell 6 originally expressing a small amount of CAR antigen with the therapeutic effect enhancer 1, was transplanted into a mouse, then the CAR-T cell 8 was further administered to the mouse, and the proliferation rate of the costimulatory factor-expressing tumor cell 7 was measured over time.

### - Preparation of tumor cell

The tumor cells used in the animal tests A to D are all Rh30 as in Example 1, and these Rh30 express a small amount of CAR antigen. Here, as described above, in the animal tests B and D, Rh30 into which a gene encoding costimulatory factors was introduced was transplanted into a mouse, and the costimulatory factors to be introduced were CD80 and 4-1BBL. The tumor cells were prepared using a DMEM medium supplemented with 10% fetal bovine serum in an incubator having 5% CO₂ atmosphere at 37°C.

### - Preparation of CAR-T cell

The CAR-T cell used in the animal tests A to D is an EphB4 CAR-T cell as in Example 1. That is, an operation of introducing the CAR gene into PBMC was performed to produce a cell population containing the CAR-T cell. The PBMC was placed in an incubator having 5% CO₂ atmosphere at 37°C with ALyS705 (containing IL7: 10 ng/mL, IL15: 5 ng/mL) supplemented with 5% artificial serum (Cell Science & Technology Institute, Inc.), and was cultured. The CAR-T cell was produced by a piggyBAC method using a DNA transferase derived from a moth. That is, a plasmid DNA for CAR expression and a plasmid DNA for piggyBAC expression were introduced into the PBMC by an electroporation method to prepare the CAR-T cell.

### - Tumor cell transplantation and administration of CAR-T cell

Animals transplanted with the respective tumor cells in the animal tests A to D are NSG (registered trademark) mice. Specifically, the respectively prepared tumor cells (2 × 10⁶ cells/0.1 mL) were subcutaneously administered to mice to prepare tumor-bearing mice. In the animal tests C and D, 0.2 mL of a solution containing the EphB4 CAR-T cells (10 x 10⁶ cells) was administered by subcutaneous injection 7 days after transplantation of the tumor cells. In the animal tests A and B which are control tests, 0.2 mL of a phosphate buffer (HEPES) was administered via a tail vein 7 days after transplantation of the tumor cells.

### - Measurement of proliferation rate of tumor cell

During the animal tests A to D, the volume of tumor cell was measured over time as an indicator of the proliferation rate of the tumor cell. The volume of tumor cell was calculated by measuring a tumor diameter with a caliper over time and calculating (short diameter × short diameter × long diameter)/2. All mice were euthanized when their tumor size reached 2000 mm³.

### - Results

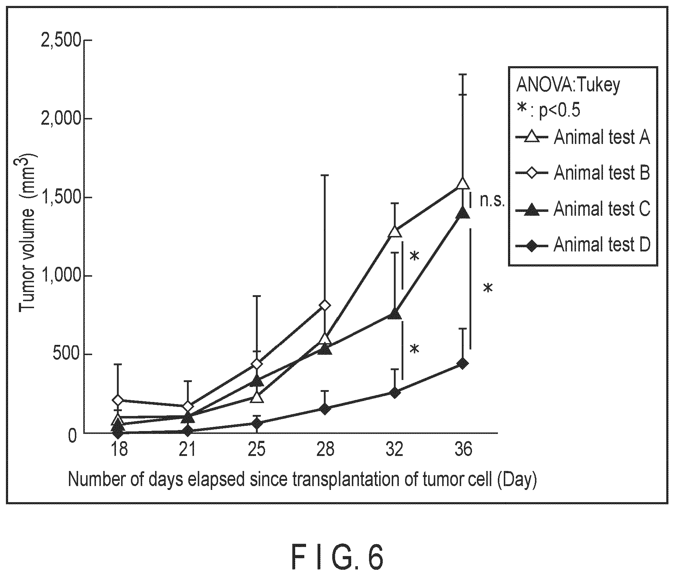

The experimental results of Example 2 are illustrated in FIG. 6. In FIG. 6, the vertical axis represents the tumor volume, and the horizontal axis represents the number of days elapsed since transplantation of the tumor cell. From the results of the animal test A, it can be confirmed that the tumor volume increases over time. In addition, in the animal test B, breeding was stopped on Day 28 due to the death of the mouse, but an increase tendency in the tumor volume similar to that in the animal test A was observed, and thus, it is understood that the expression of a costimulatory factor alone does not affect the proliferation rate of the tumor cell.

In the animal test C, a tumor volume which is about half the size of the tumor volume in the animal test A was observed on Day 32 after transplantation of the tumor cells, but the tumor volume on Day 36 reached the same level as that in the animal test A. On the other hand, it is understood that although an increase in the tumor volume was also observed in the animal test D, the tumor volume was generally about 25% of those in the animal tests B to D on and after Day 25 after transplantation of the tumor cells. Accordingly, it was revealed that the cytotoxic effect of the CAR-T cell was significantly enhanced when not only the CAR antigen but also the costimulatory factor was expressed, demonstrating superior efficacy.

### Example 3. Animal test for confirmation of expression of costimulatory factor in tumor cell administered with therapeutic effect enhancer

In order to confirm the enhancement of the expression of the CAR antigen in the tumor cell by the therapeutic effect enhancer and the expression of the costimulatory factor, the following animal test E was performed.

### - Preparation of tumor cell

The tumor cell used in the animal test E is Rh30 as in Examples 1 and 2. Rh30 was prepared using a DMEM medium supplemented with 10% fetal bovine serum in an incubator having 5% CO₂ atmosphere at 37°C.

### - Preparation of therapeutic effect enhancer

As the nucleic acids to be encapsulated in lipid particles, mRNA encoding the extracellular domain of the CAR antigen EphB4 and mRNA encoding CD80 and 4-1BBL which are costimulatory factors were used in the animal test E as in the cell test C of Example 1 and the animal test D of Example 2. A solution containing each mRNA and an lipid-dissolved ethanol solution having a lipid composition described in Table 1 above were mixed, then further mixed with 10mM HEPES (pH7.3), and washed and concentrated by centrifugal ultrafiltration to obtain a solution containing lipid particles (that is, the therapeutic effect enhancer of the first arrangement).

### - Tumor cell transplantation and administration of therapeutic effect enhancer

NSG mice were transplanted with tumor cells as in the animal tests A to D of Example 2. Specifically, the prepared tumor cells (2 × 10⁶ cells/0.1 mL) were subcutaneously administered to mice to prepare tumor-bearing mice. When fixing of the tumor was confirmed, 50 µL of the therapeutic effect enhancer (200 ng mRNA/µL) was administered into the tumor. Three days after the administration of the therapeutic effect enhancer, the tumor was excised and fixed with formalin, and then paraffin sections were prepared. Immunohistological staining (IHC) was performed on the prepared paraffin sections using an anti-EphB4 antibody as an anti-CAR antigen, and an anti-CD 80 antibody and an anti-4-1BBL antibody as anti-costimulatory factor antibodies. IHC results for each antibody were photographed with Vectra (registered trademark) 3, and the diaminobenzidine (DAB) positive rate was analyzed with an image analysis software, inForm.

### - Results

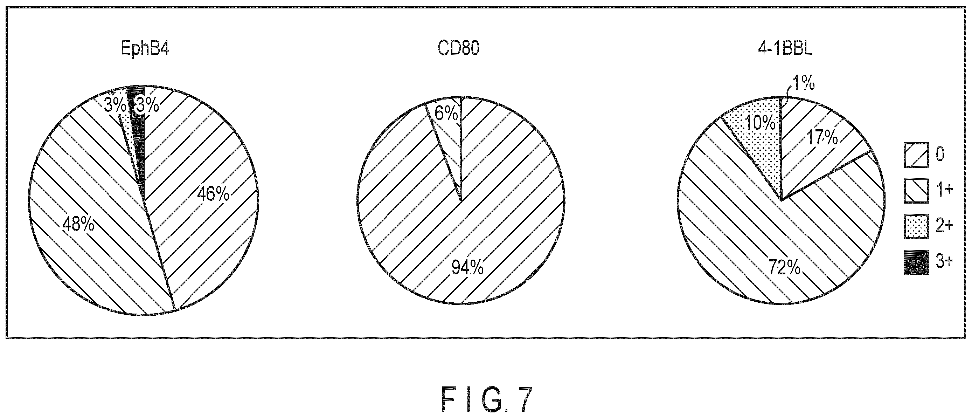

The experimental results are illustrated in FIG. 7. FIG. 7 is a circular graph illustrating a DAB positive rate (intensity) of EphB4, a circular graph illustrating a DAB positive rate (intensity) of CD80, and a circular graph illustrating a DAB positive rate (intensity) of 4-1BBL. Note that "0", "+1", "+2", and "+3" in the circular graph represent the magnitude of the detected intensity, which means that the intensity is higher in this order.

Referring to FIG. 7, the DAB positive rate of CD80 was very low, while EphB4 and 4-1BBL had high DAB positive rates. This result indicates that both the CAR antigen and the costimulatory factor contained in the therapeutic effect enhancer were expressed in the tumor to which the therapeutic effect enhancer was administered in the animal test E. Therefore, it was confirmed that the therapeutic effect enhancer of the first arrangement introduces the costimulatory factor into the tumor cell and expresses the costimulatory factory.

### Example 4. Animal tests for confirmation of tumor cell cytotoxic effect and persistence of therapeutic effect enhancer

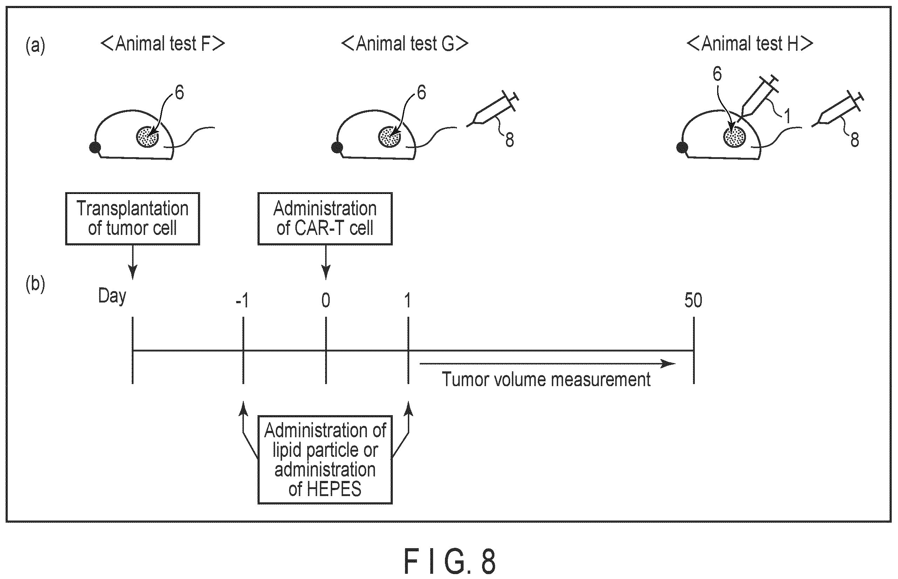

In order to confirm the tumor cell cytotoxic effect by the therapeutic effect enhancer and the persistence thereof, the following animal tests F to H were performed. First, the experimental designs of the animal tests F to H will be briefly described with reference to FIG. 8.

As illustrated in FIG. 8, the animal test F is a comparative example for confirming the proliferation rate of untreated tumor cells. The animal test F was conducted using a procedure almost identical to that of the animal test A of Example 2, except for the period between tumor cell transplantation and HEPES administration, as well as the number of HEPES administrations.

As illustrated in FIG. 8, the animal test G is a comparative example for confirming the cytotoxic effect of the CAR-T cell 8 on the tumor cell 6 when the therapeutic effect enhancer of the present application is not used. Specifically, the tumor cell 6 originally expressing a small amount of CAR antigen was transplanted into a mouse, and then, the CAR-T cell 8 was further administered to the mouse to measure the proliferation rate of the tumor cell over time.

As illustrated in FIG. 8, the animal test H is an example for confirming the cytotoxic effect of the CAR-T cell 8 on the tumor cell 6 when the therapeutic effect enhancer 1 of the first arrangement was used. Specifically, the tumor cell 6 originally expressing a small amount of CAR antigen was transplanted into a mouse, then the therapeutic effect enhancer 1 was administered to the mouse, then the CAR-T cell 8 was administered, and the therapeutic effect enhancer 1 was further administered at a later date, and the proliferation rate of the tumor cell 6 was measured over time.

### - Preparation of tumor cell

The tumor cell used in the animal tests F to H is Rh30 as in Examples 1, 2 and 3. Specifically, Rh30 was prepared using a DMEM medium supplemented with 10% fetal bovine serum in an incubator having 5% CO₂ atmosphere at 37°C.

### - Preparation of CAR-T cell

The CAR-T cell used in the animal tests F to H is an EphB4 CAR-T cell as in Examples 1 and 2. That is, an operation of introducing the CAR gene into PBMC was performed to produce a cell population containing the CAR-T cell. The PBMC was placed in an incubator having 5% CO₂ atmosphere at 37°C with ALyS705 (containing IL7: 10 ng/mL, IL15: 5 ng/mL) supplemented with 5% artificial serum (Cell Science & Technology Institute, Inc.), and was cultured. The CAR-T cell was produced by a piggyBAC method using a DNA transferase derived from a moth. That is, a plasmid DNA for CAR expression and a plasmid DNA for piggyBAC expression were introduced into the PBMC by an electroporation method to prepare the CAR-T cell.

### - Preparation of therapeutic effect enhancer

As the nucleic acids to be encapsulated in lipid particles, mRNA encoding the extracellular domain of the CAR antigen EphB4 and mRNA encoding CD80 and 4-1BBL which are costimulatory factors were used in the animal test H as in the cell test C of Example 1 and the animal test D of Example 2. A solution containing each mRNA and an lipid-dissolved ethanol solution having a lipid composition described in Table 1 above were mixed, then further mixed with 10mM HEPES (pH7.3), and washed and concentrated by centrifugal ultrafiltration to obtain a solution containing lipid particles (that is, the therapeutic effect enhancer of the first arrangement).

### - Tumor cell transplantation and administration of CAR-T cell

In the animal tests F to H, NSG mice were also transplanted with tumor cells as in the animal tests A to D of Example 2. Specifically, the prepared tumor cells (2 × 10⁶ cells/0.1 mL) were subcutaneously administered to mice to prepare tumor-bearing mice. In the animal tests F and G which are comparative examples, when the tumor became large to some extent, 0.2 mL of a solution containing HEPES or EphB4 CAR-T cells (10 × 10⁶ cells/mL) was administered via a tail vein.

### - Administration of CAR-T cell and therapeutic effect enhancer

In the animal test H, 50 µL of the therapeutic effect enhancer (200 ng mRNA/µL) was administered into the tumor on the day before administration of HEPES or CAR-T cell in the animal tests F and G. On the next day, 0.2 mL of a solution containing EphB4 CAR-T cells (10 × 10⁶ cells/mL) was administered via a tail vein, and on the day after that, 50 µL of the therapeutic effect enhancer (200 ng mRNA/µL) was administered into the tumor again. The time courses of the above animal tests F to H are illustrated in FIG. 8.

### - Measurement of proliferation rate of tumor cell

In the animal tests F to H, as in the animal tests A to D, the volume of tumor cell was measured over time as an index of the proliferation rate of the tumor cell during a rearing period. The volume of tumor cell was calculated by measuring a tumor diameter with a caliper over time and calculating (short diameter × short diameter × long diameter)/2. All mice were euthanized when their tumor size reached 2000 mm³.

### - Results

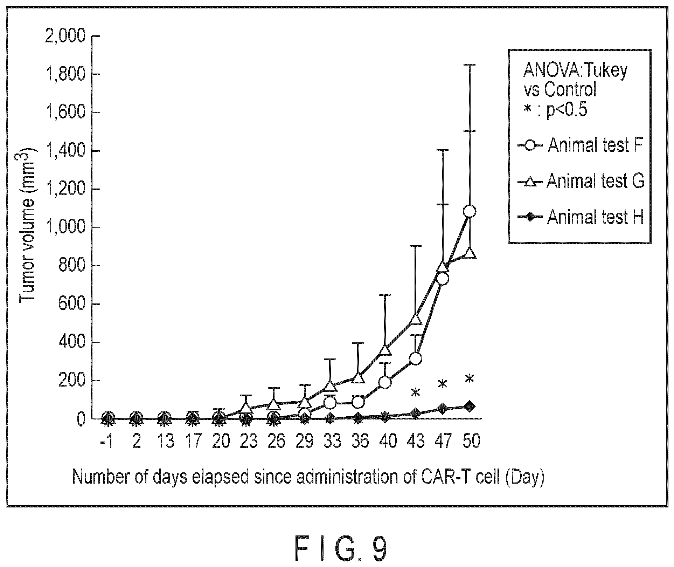

The experimental results of Example 4 are illustrated in FIG. 9. In FIG. 9, the vertical axis represents the tumor volume, and the horizontal axis represents the number of days elapsed after administration of the CAR-T cell. From the results of the animal test F, it can be confirmed that the tumor volume increases over time from around Day 23 after the administration of the CAR-T cell. In the animal test G, the tumor volume increased over time from around Day 29 to Day 33 after administration of the CAR-T cell, and the tumor volume was about half of that in the animal test F until Day 43, but the same tumor volume as that in the animal test F was observed after Day 47. From this result, it can be seen that administration of the CAR-T cell delayed the start of proliferation of the tumor cell, but ultimately the cytotoxic effect of the CAR-T cell was limited.

In the animal test H, the tumor volume increased over time from around Day 43 to Day 47 after administration of the CAR-T cell, but even at the time point of Day 50, the tumor volume was very small compared to those in the animal tests F and G. Therefore, it was revealed that the cytotoxic effect of the CAR-T cell was highly exerted through the administration of the therapeutic effect enhancer of the present arrangement.

While certain arrangements have been described, these arrangements have been presented by way of example only, and are not intended to limit the scope of the claims. Indeed, the therapeutic effect enhancer described herein may be embodied in a variety of other forms; furthermore, various omissions, substitutions and changes in the form of the therapeutic effect enhancer described herein may be made.

The arrangements as described above include clauses below.

### Clause 1

A therapeutic effect enhancer for promoting a therapeutic effect of a CAR-T cell therapy, the therapeutic effect enhancer characterized by comprising: a nucleic acid that contains a gene encoding a target antigen of a CAR; and a nucleic acid that contains a gene encoding a costimulatory factor.

### Clause 2

The therapeutic effect enhancer according to clause 1, characterized by further comprising a lipid particle,
wherein the lipid particle encapsulates the nucleic acid which contains the gene encoding the target antigen of the CAR and the nucleic acid which contains the gene encoding the costimulatory factor in a lipid membrane.

### Clause 3.

The therapeutic effect enhancer according to clause 2, characterized in that the lipid membrane contains a lipid compound represented by formula (1-01) and/or a lipid compound represented by formula (1-02) below.

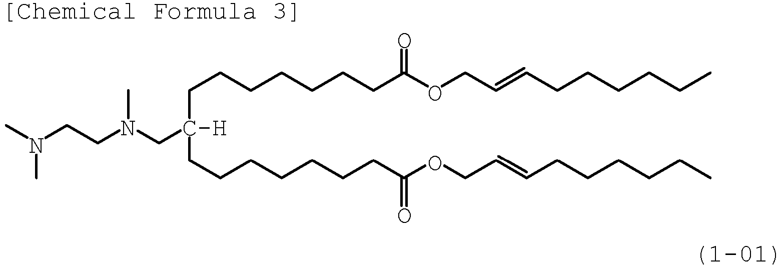

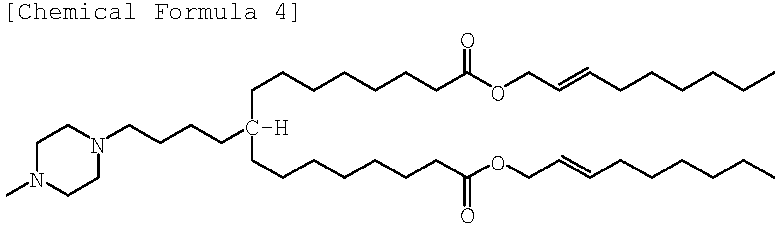

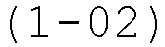

### Clause 4.

The therapeutic effect enhancer according to clause 3, characterized in that the lipid membrane further contains at least a lipid compound selected from a group consisting of a cationic lipid, a neutral lipid, an aggregation-reducing lipid, and a structure-forming lipid.

### Clause 5.

The therapeutic effect enhancer according to clause 1,
the therapeutic effect enhancer characterized by further comprising two kinds of lipid particle,
wherein one kind of lipid particle encapsulates the nucleic acid which contains the gene encoding the target antigen of the CAR, and
another kind of lipid particle encapsulates the nucleic acid which contains the gene encoding the costimulatory factor.

### Clause 6.

The therapeutic effect enhancer according to clause 5,
characterized in that the one kind of lipid particle and the another kind of lipid particle each includes a lipid membrane which contains a lipid compound represented by formula (1-01) and/or a lipid compound represented by formula (1-02) below, and
a lipid composition of the lipid membrane of the one kind of lipid particle and a lipid composition of the lipid membrane of the another kind of lipid particle are identical or different.

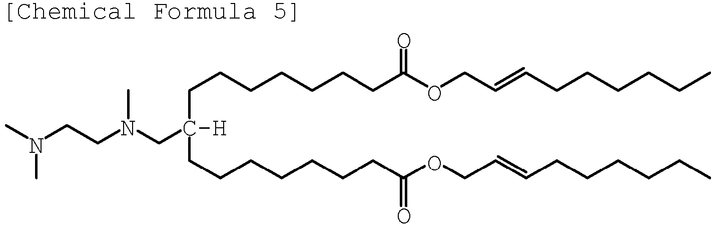

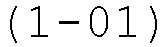

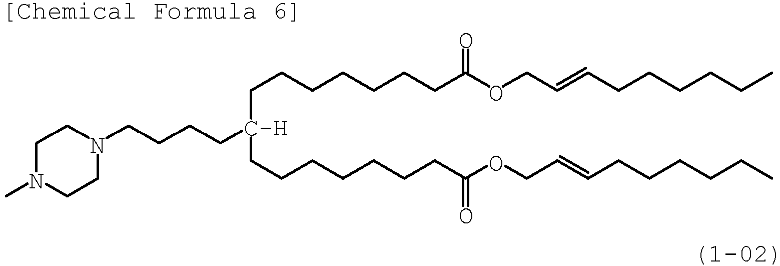

### Clause 7.

The therapeutic effect enhancer according to clause 6, characterized in that the lipid membrane of each of the one kind of lipid particle and the another kind of lipid particle further contains at least a lipid compound selected from a group consisting of a cationic lipid, a neutral lipid, an aggregation-reducing lipid, and a structure-forming lipid.

### Clause 8.

The therapeutic effect enhancer according to clause 1, characterized in that the nucleic acid which contains the gene encoding the costimulatory factor is a full length or a part of at least a nucleic acid selected from a group consisting of 4-1BBL, CD80, CD86, ICOSL, and OX40L.

### Clause 9.

The therapeutic effect enhancer according to clause 8, characterized in that the nucleic acid which contains the gene encoding the costimulatory factor is at least a nucleic acid selected from a group consisting of DNA, mRNA, plasmid, and polynucleotide.

### Clause 10.

The therapeutic effect enhancer according to clause 2, characterized by further comprising a storage stabilizer for the lipid particle.

### Clause 11.

The therapeutic effect enhancer according to clause 5, characterized by further comprising a storage stabilizer for the one kind of lipid particle and the another kind of lipid particle.

### Clause 12.

The therapeutic effect enhancer according to clause 10 or 11, characterized in that the storage stabilizer is a pH adjuster, a buffering agent, a tonicity adjuster, or a cryoprotectant.

## Claims

1. A therapeutic effect enhancer for promoting a therapeutic effect of a CAR-T cell therapy, the therapeutic effect enhancer **characterized by** comprising: a nucleic acid that contains a gene encoding a target antigen of a CAR; and a nucleic acid that contains a gene encoding a costimulatory factor.

2. The therapeutic effect enhancer according to claim 1, **characterized by** further comprising a lipid particle,
wherein the lipid particle encapsulates the nucleic acid which contains the gene encoding the target antigen of the CAR and the nucleic acid which contains the gene encoding the costimulatory factor in a lipid membrane.

3. The therapeutic effect enhancer according to claim 2, **characterized in that** the lipid membrane contains a lipid compound represented by formula (1-01) and/or a lipid compound represented by formula (1-02) below.

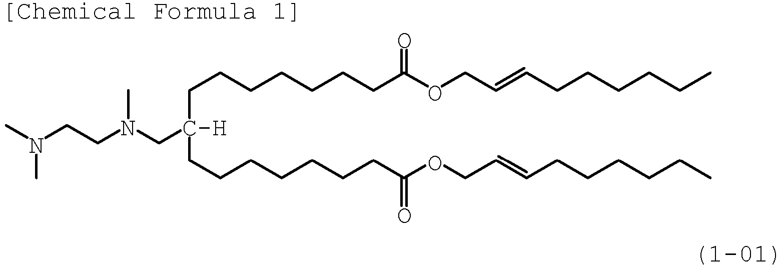

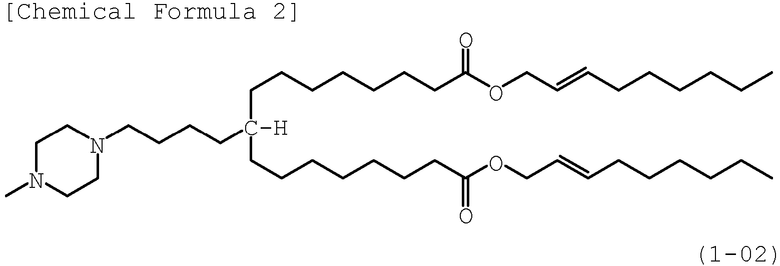

4. The therapeutic effect enhancer according to claim 3, **characterized in that** the lipid membrane further contains at least a lipid compound selected from a group consisting of a cationic lipid, a neutral lipid, an aggregation-reducing lipid, and a structure-forming lipid.

5. The therapeutic effect enhancer according to claim 1,
the therapeutic effect enhancer **characterized by** further comprising two kinds of lipid particle,
wherein one kind of lipid particle encapsulates the nucleic acid which contains the gene encoding the target antigen of the CAR, and
another kind of lipid particle encapsulates the nucleic acid which contains the gene encoding the costimulatory factor.

6. The therapeutic effect enhancer according to claim 5,
**characterized in that** the one kind of lipid particle and the another kind of lipid particle each includes a lipid membrane which contains a lipid compound represented by formula (1-01) and/or a lipid compound represented by formula (1-02) below, and
a lipid composition of the lipid membrane of the one kind of lipid particle and a lipid composition of the lipid membrane of the another kind of lipid particle are identical or different.

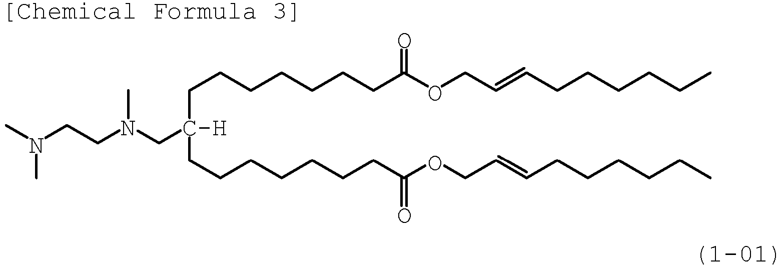

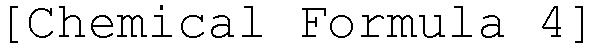

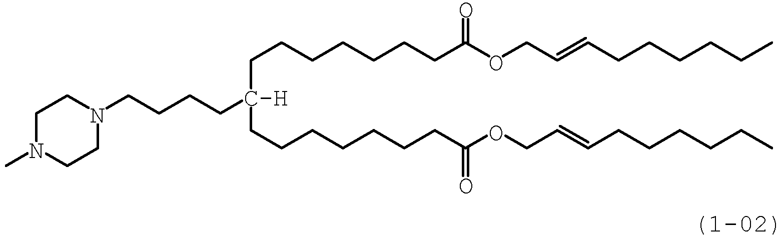

7. The therapeutic effect enhancer according to claim 6, **characterized in that** the lipid membrane of each of the one kind of lipid particle and the another kind of lipid particle further contains at least a lipid compound selected from a group consisting of a cationic lipid, a neutral lipid, an aggregation-reducing lipid, and a structure-forming lipid.

8. The therapeutic effect enhancer according to claim 1, **characterized in that** the nucleic acid which contains the gene encoding the costimulatory factor is a full length or a part of at least a nucleic acid selected from a group consisting of 4-1BBL, CD80, CD86, ICOSL, and OX40L.

9. The therapeutic effect enhancer according to claim 8, **characterized in that** the nucleic acid which contains the gene encoding the costimulatory factor is at least a nucleic acid selected from a group consisting of DNA, mRNA, plasmid, and polynucleotide.

10. The therapeutic effect enhancer according to claim 2, **characterized by** further comprising a storage stabilizer for the lipid particle.

11. The therapeutic effect enhancer according to claim 5, **characterized by** further comprising a storage stabilizer for the one kind of lipid particle and the another kind of lipid particle.

12. The therapeutic effect enhancer according to claim 10 or 11, **characterized in that** the storage stabilizer is a pH adjuster, a buffering agent, a tonicity adjuster, or a cryoprotectant.
